# EUROPÄISCHE PATENTANMELDUNG

(11) **EP 1 338 293 A1**
(43) Veröffentlichungstag der Anmeldung: **27.08.2003**
(21) Anmeldenummer: 03002978.9
(22) Anmeldetag: 11.02.2003
(51) Int. Cl.: A61L 31/02, A61L 31/14

(54) **Verfahren zur Herstellung von bioresorbierbaren röhrenförmigen Metallimplantaten**

(30) Priorität: 20.02.2002 DE 10207161
(71) Anmelder: Universität Hannover, 30167 Hannover (DE)
(72) Erfinder: Kaese, Volker, 30161 Hannover (DE); Haferkamp, Heinz, 30826 Garbsen (DE); Pinkvos, Arne, 30851 Langenhagen (DE); Niemeyer, Matthias, 30163 Hannover (DE); Bach, Friedrich-Wilhelm, 30916 Isernhagen (DE)
(74) Vertreter: Scheffler, Jörg

(57) **Zusammenfassung**

Die Erfindung betrifft ein Verfahren zur Herstellung von Implantaten aus bioresorbierbarem Metall, insbesondere aus Magnesiumlegierungen oder Zinklegierungen. Hierdurch werden die Werkstoffeigenschaften des Magnesiums oder des Zinks verändert und dadurch die Verarbeitungs- und Gebrauchseigenschaften verbessert. Durch die Kombination von Verfahrensschritten zur Einstellung des Werkstoffzustands und die anschließende mechanische Bearbeitung ist es möglich dünnwandige röhrenförmige Implantate, insbesondere Gefäßstützen, aus Magnesium- oder Zinklegierungen herzustellen, die problemlos verformbar sind, ohne dass eine Bruchgefahr beim Implantieren besteht.

## Beschreibung

Die Erfindung betrifft ein Verfahren zur Herstellung von Implantaten aus bioresorbierbarem Metall, insbesondere aus Magnesiumlegierungen oder Zinklegierungen.

Verfahren zur Herstellung von Implantaten aus bioresorbierbarem Metall der eingangs genannten Art sind in der Praxis durch offenkundige Vorbenutzung bekannt und zählen dadurch zum Stand der Technik. Mittels dieser Verfahren werden beispielsweise Schrauben oder Nägel aus Magnesiumlegierungen für die Fixierung von Knochen bei Brüchen hergestellt. Implantate aus bioresorbierbarem Metall zeichnen sich dadurch aus, dass sie im menschlichen Körper korrodieren und sich nahezu rückstandsfrei auflösen. Durch die Art der Legierungsbestandteile bzw. die Menge der Legierungsbestandteile ist die Zeitspanne, in der sich die Implantate komplett auflösen, einstellbar. Dadurch müssen die nach der Heilung der Knochenbrüche überflüssigen Nägel oder Schrauben nicht mehr operativ entfernt werden, was eine enorme Erleichterung für den Genesungsprozess des Patienten, eine wesentliche Arbeitserleichterung und Zeitersparnis für den Arzt und dadurch eine erhebliche Reduzierung der Kosten im Gesundheitswesen bedeutet.

Durch die werkstofflichen Eigenschaften, wie beispielsweise die hexagonale Gitterstruktur und die damit verbundene geringe Duktilität bei Raumtemperatur, sind der Verarbeitung dieser bioresorbierbaren Metalle jedoch Grenzen gesetzt, insbesondere ist der Einsatz solcher Implantate auf Anwendungen beschränkt, bei denen mögliche Bruchstücke nicht in die Blutbahn des Patienten gelangen können. Hierdurch wird die Einsatzmöglichkeit von Magnesium- oder Zinklegierungen im Wesentlichen auf Implantate für Knochenbrüche beschränkt.

Vor diesem Hintergrund liegt der Erfindung die Aufgabe zugrunde, ein Verfahren zu schaffen, mit dem die Einsatzmöglichkeit von bioresorbierbaren Metallen, wie beispielsweise Magnesiumlegierungen oder Zinklegierungen, für die Herstellung von Implantaten erweitert wird.

Diese Aufgabe wird erfindungsgemäß mit einem Verfahren gemäß den Merkmalen des Patentanspruchs 1 gelöst. Die weitere Ausgestaltung der Erfindung ist den Unteransprüchen zu entnehmen.

Erfindungsgemäß ist ein Verfahren vorgesehen, bei welchem aus dem bioresorbierbaren Metall durch Gießen und eine Wärmebehandlung, insbesondere durch Homogenisieren, und durch eine anschließende thermomechanische Behandlung, insbesondere durch Strangpressen, ein bolzenförmiges Halbzeug hergestellt, danach durch ein Trennverfahren in Längsrichtung in zumindest zwei Teilstücke geteilt und das jeweilige Teilstück schließlich zur Herstellung eines röhrenförmigen Implantats, insbesondere einer Gefäßstütze, spanend bearbeitet wird. Hierdurch werden die Werkstoffeigenschaften des Magnesiums oder des Zinks verändert und dadurch die Verarbeitungs- und Gebrauchseigenschaften verbessert, so dass ein Abbrechen von Teilstücken weitgehend ausgeschlossen werden kann. Durch die Kombination der vorgeschalteten Prozesse zur Einstellung des Werkstoffzustands und die anschließende mechanische Bearbeitung ist es möglich, dünnwandige röhrenförmige Implantate, beispielsweise cardiovaskuläre Gefäßstützen, aus Magnesium- oder Zinklegierungen herzustellen. Diese röhrenförmigen Implantate, sogenannte Stents, stützen Gefäße, beispielsweise Arterien, im menschlichen Körper von innen ab. Durch dieses Verfahren wird gewährleistet, dass die röhrenförmigen Implantate eine ausreichende mechanische Festigkeit bei einer gleichmäßigen kontrollierbaren Korrosion über den gesamten Wandungsquerschnitt aufweisen. Dabei sind die so geschaffenen Implantate verformbar, ohne dass bei einer Belastung ein Bruch zu befürchten ist. Dadurch werden mögliche Verletzungen der Arterien durch brechende Implantate oder durch abgebrochene und weggespülte Ausbrüche des Implantats ebenso vermieden, wie ein unerwünschtes Verschieben des gesamten Implantats.

Durch das Gießen und die Wärmebehandlung wird eine homogene Verteilung des Magnesiums oder des Zinks und der jeweiligen Legierungsbestandteile erreicht. Beim Gießen entstehende Lunker, sowie Ausscheidungen und Verunreinigungen, können in einem Zwischenschritt abgedreht werden. Hierdurch wird gewährleistet, dass sich das röhrenförmige Implantat über den gesamten Wandungsquerschnitt gleichmäßig und einstellbar auflöst. Durch die anschließende thermomechanische Behandlung, beispielsweise durch warmes Strangpressen, wird durch einen hohen Umformgrad eine Kornfeinung erzielt. Hierdurch wird die benötigte mechanische Festigkeit des bolzenförmigen Halbzeugs erreicht, da über den Wandungsquerschnitt mehrere Körner mechanisch tragen. Durch die ersten beiden Verfahrensschritte werden gleichmäßige Werkstoffeigenschaften über die gesamte Länge des bolzenförmigen Halbzeugs eingestellt. Durch das Strangpressen entstehen über den Querschnitt des bolzenförmigen Halbzeugs Bereiche mit unterschiedlichen Werkstoffeigenschaften, da beim Strangpressen in den Außenbereichen eine größere Umformung erfolgt als im Innenbereich. Aus diesem Grund wird das bolzenförmige Halbzeug anschließend entlang seiner Längsachse in zumindest zwei Teilstücke geteilt. Hierdurch ist es möglich, für die anschließende spanende Bearbeitung Teilstücke aus den Bereichen hoher Umformgrade und somit hoher Kornfeinung zu entnehmen. Durch die spanende Bearbeitung wird anschließend der Innen- und der Außendurchmesser des röhrenförmigen Implantats hergestellt.

Als besonders praxisnah erweist sich, dass das bolzenförmige Halbzeug in drei oder vier Teilstücke geteilt wird. Hierdurch können die Teilstücke für die spanende Bearbeitung in ein Drei- oder Vierbackenfutter eingespannt werden.

Eine besonders zweckmäßige Weiterbildung des Verfahrens wird dadurch erreicht, dass die spanende Bearbeitung ohne den Einsatz von Kühlschmiermitteln durchgeführt wird. Durch die Trockenbearbeitung wird ein Korrosionsangriff an den, durch die spanende Bearbeitung entstandenen Wandungen vermieden. Für die Trockenbearbeitung sollten die Werkzeuge ausreichend scharf sein.

Eine besonders vorteilhafte Ausgestaltung des Verfahrens wird dadurch erreicht, dass die Verfahrensschritte automatisiert durchgeführt werden. Hierdurch werden Verzögerungen im Fertigungsprozess ebenso vermieden, wie mögliche Fehlerquellen durch einen manuellen Eingriff. Dadurch wird ein wirtschaftliches Verfahren zur Herstellung von röhrenförmigen Implantaten geschaffen.

Dabei ist es besonders einfach, wenn ein Innendurchmesser des röhrenförmigen Implantats durch Bohren und ein Außendurchmesser durch Drehen hergestellt wird. Hierzu wird das jeweilige Teilstück mit einem vorbestimmten Soll-Innendurchmesser angebohrt und die Außenfläche des jeweiligen Teilstücks wird bis zum gewünschten Soll-Außendurchmesser abgedreht.

Eine andere besonders zweckmäßige Weiterbildung des Verfahrens wird dadurch erreicht, dass die spanende Bearbeitung zur Herstellung des Innendurchmessers und des Außendurchmessers des röhrenförmigen Implantats simultan erfolgt. Hierdurch lassen sich Implantate mit besonders gleichmäßigen, dünnen Wandungen mit Innendurchmessern von ca. 1,8 mm und Außendurchmessern von ca. 2,0 mm bei einer Länge von ca. 15,0 mm herstellen. Diese gleichmäßigen Wandungen sind insbesondere bei radial verformbaren Implantaten wichtig, um eine gleichmäßige Belastung und somit Dehnung zu erzielen.

Eine weitere besonders vorteilhafte Abwandlung des Verfahrens wird dadurch erreicht, dass nach der spanenden Bearbeitung eine Feinbearbeitung durchgeführt wird. Hierdurch werden mechanisch nachteilige Beeinträchtigungen der Oberflächenbeschaffenheit, beispielsweise durch Kerben, in dem röhrenförmigen Implantat vermieden.

Eine weitere besonders zweckmäßige Ausgestaltung des Verfahrens wird dadurch erreicht, dass ein athermisches Trennverfahren eingesetzt wird. Hierdurch werden Veränderungen des Gefüges und ein Ausdiffundieren von niedrigschmelzenden Elementen und Legierungsbestandteilen in dem bolzenförmigen Halbzeug bzw. in den jeweiligen Teilstücken durch andernfalls bei dem Trennverfahren entstehende oder zugeführte Wärme verhindert.

Als besonders praxisnah hat sich erwiesen, dass zum Trennen ein Jet-Cutting-Verfahren eingesetzt wird. Hierdurch entsteht keine Wärme und somit wird eine Veränderung des Gefüges vermieden. Zugleich können dadurch feine Strukturen mit hoher Genauigkeit hergestellt werden.

Eine weitere besonders einfache Abwandlung wird dadurch erreicht, dass bei dem Trennverfahren ein Sägeblatt mit verschränkten Zähnen eingesetzt wird. Hierdurch wird die Wärmeentwicklung reduziert. Das Sägeblatt kann zusätzlich kühlbar sein.

Eine andere besonders zweckmäßige Weiterbildung wird dadurch erreicht, dass nach dem Trennen des Halbzeuges eine Wärmebehandlung, insbesondere ein Auslagern, durchgeführt wird. Hierdurch kann das mechanische Eigenschaftsprofil gezielt verändert werden.

Eine weitere besonders vorteilhafte Ausgestaltung des Verfahrens wird dadurch erreicht, dass das bolzenförmige Halbzeug derart geteilt wird, dass bei der Herstellung des röhrenförmigen Implantats ein zentraler, die Mittelachse des bolzenförmigen Halbzeugs einschließender Bereich ausgespart wird. Hierdurch wird verhindert, dass die sogenannte Nullseele des bolzenförmigen Halbzeugs Bestandteil des röhrenförmigen Implantats ist. Die Nullseele ist der Bereich mit dem geringsten Umformgrad und somit der geringsten Kornfeinung.

## Patentansprüche

1. Verfahren zur Herstellung von Implantaten aus bioresorbierbarem Metall, insbesondere aus Magnesiumlegierungen oder Zinklegierungen, **dadurch gekennzeichnet, dass** aus dem bioresorbierbaren Metall durch Gießen und eine Wärmebehandlung, insbesondere durch Homogenisieren, und durch eine anschließende thermomechanische Behandlung, insbesondere durch Strangpressen, ein bolzenförmiges Halbzeug hergestellt, danach durch ein Trennverfahren in Längsrichtung in zumindest zwei Teilstücke geteilt und das jeweilige Teilstück schließlich zur Herstellung eines röhrenförmigen Implantats, insbesondere einer Gefäßstütze, spanend bearbeitet wird.

2. Verfahren nach Anspruch 1, **dadurch gekennzeichnet, dass** das bolzenförmige Halbzeug in drei oder vier Teilstücke geteilt wird.

3. Verfahren nach Anspruch 1 oder 2, **dadurch gekennzeichnet, dass** die spanende Bearbeitung ohne den Einsatz von Kühlschmiermitteln durchgeführt wird.

4. Verfahren nach zumindest einem der vorhergehenden Ansprüche, **dadurch gekennzeichnet, dass** die Verfahrensschritte automatisiert durchgeführt werden.

5. Verfahren nach zumindest einem der vorhergehenden Ansprüche, **dadurch gekennzeichnet, dass** ein Innendurchmesser des röhrenförmigen Implantats durch Bohren und ein Auβendurchmesser durch Drehen hergestellt wird.

6. Verfahren nach zumindest einem der vorhergehenden Ansprüche, **dadurch gekennzeichnet, dass** die spanende Bearbeitung zur Herstellung des Innendurchmessers und des Außendurchmessers des röhrenförmigen Implantats simultan erfolgt.

7. Verfahren nach zumindest einem der vorhergehenden Ansprüche, **dadurch gekennzeichnet, dass** nach der spanenden Bearbeitung eine Feinbearbeitung durchgeführt wird.

8. Verfahren nach zumindest einem der vorhergehenden Ansprüche, **dadurch gekennzeichnet, dass** ein athermisches Trennverfahren eingesetzt wird.

9. Verfahren nach Anspruch 8, **dadurch gekennzeichnet, dass** zum Trennen ein Jet-Cutting-Verfahren eingesetzt wird.

10. Verfahren nach zumindest einem der Ansprüche 1-8, **dadurch gekennzeichnet, dass** bei dem Trennverfahren ein Sägeblatt mit verschränkten Zähnen eingesetzt wird.

11. Verfahren nach zumindest einem der vorhergehenden Ansprüche, **dadurch gekennzeichnet, dass** nach dem Trennen des Halbzeuges eine Wärmebehandlung, insbesondere ein Auslagern, durchgeführt wird.

12. Verfahren nach zumindest einem der vorhergehenden Ansprüche, **dadurch gekennzeichnet, dass** das bolzenförmige Halbzeug derart geteilt wird, dass bei der Herstellung des röhrenförmigen Implantats ein zentraler, die Mittelachse des bolzenförmigen Halbzeugs einschließender Bereich ausgespart wird.
